# EUROPEAN PATENT APPLICATION

(11) **EP 1 582 219 A1**
(43) Date of publication of application: **05.10.2005**
(21) Application number: 03814541.3
(22) Date of filing: 28.11.2003
(51) Int. Cl.: A61K 45/00, A61K 31/16, A61K 31/336, A61P 35/04

(54) **CONNEXIN 26 INHIBITORS AND CANCER METASTASIS INHIBITORS**

(30) Priority: 29.11.2002 JP 2002349155
(71) Applicant: Japan Science and Technology Agency, Saitama 332-0012 (JP)
(72) Inventor: KITA, Yasuyuki, Kashiwara-shi, Osaka 582-0015 (JP); NOJIMA, Hiroshi, Minoo-shi, Osaka 562-0031 (JP); ITO, Akihiko, Toyonaka-shi, Osaka 560-0085 (JP); MORITA, Nobuyoshi, Suita-shi, Osaka 565-0842 (JP)
(74) Representative: Müller, Frithjof E.
(86) International application number: PCT/JP2003/015243
(87) International publication number: WO 2004/060398

(57) **Abstract**

A connexin 26 inhibitor and a cancer metastasis inhibitor have a cis-oxirane structure and a terminal amide structure as represented by the following general formula (1): 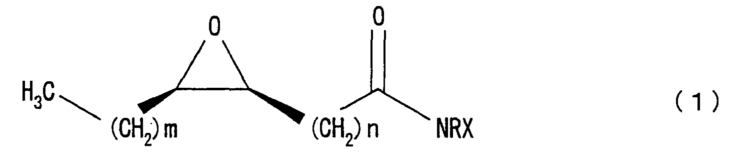 where R is a hydrogen atom or a hydrocarbon group, X is one of a hydrogen atom, a methansulfonyl group, an ethansulfonyl group, an acetyl group, a trifluoroacetyl group, a hydroxyl group, an alkoxy group and an amino group; m is an integer of from 4 to 10; and n is an integer of from 4 to 7..This compound is a novel cancer metastasis inhibitor which specifically inhibits the function of connexin 26.

## Description

### TECHNICAL FIELD

The present invention relates to a connexin 26 inhibitor for specifically inhibiting a function of connexin 26, and a novel cancer metastasis inhibitor utilizing the connexin 26 inhibitor.

### BACKGROUND ART

Connexin is a collective term of a family of connective membrane proteins. Connexin forms a gap junction. Depending on differences of its molecular weight, more than 12 types of connexin have been found. Connexin having a molecular weight of 26 kDa is termed as connexin 26, and connexin having a molecular weight of 43 kDa is termed as connexin 43.

Hexamer of connexin is termed as connexon. Connexon forms a tubular structure, which penetrates cell membranes. The gap junction is cell-to-cell connection of connexons. Through a tube formed with the connection, ions and low molecular weight proteins are transferred from a cell to a cell. This mechanism is believed to be necessary for sustaining homeostatic growth of epithelial cells.

Through basic researches for finding a cancer metastasis mechanism in genetic level, the inventors of the present invention have found that connexin 26 is closely related to cancer metastasis. Further, it has been found that inhibition of a function of connexin 26 biochemically inhibits cancer metastasis (Japanese Laid-Open Patent Publication No. 17184/2001, "*Tokukai* 2001-17184", publication date: January 23, 2001, Ito, A. et al.: J. Clin. Invest., 105:1189-1197, 2000) .

One of the well known materials inhibiting the gap junction is oleamide, which is an amide derivative of oleic acid, for example (Japanese Laid-Open Patent Publication No. 523696/2001, "*Tokuhyo* 2001-523696", December 27, 2001, Boger, D. L. et al., Proc. Nat. Acad. Sci. USA., 95:4810-4815. 1998.).

Current Cancer treatments treat cancer cells directly. Examples of the current cancer treatments are: administration of an anticancer agent, extirpation of cancer cells and cancer tissue, and radiation therapy, for example. However, because cancer cells metastasize quite quickly, cancer cells might metastasize to other tissue during cancer treatment. As a result, the treatment is prolonged and a cancer patient dies in the middle of the treatment.

Therefore, in order to improve effects of cancer treatments, it is important to inhibit cancer metastasis in addition to direct destruction of cancer through administration of anticancer agents and extirpation of the cancer tissue.

However, researches and development of cancer metastasis inhibitors based on the cancer metastasis mechanism have been proceeded with difficulties, and no cancer metastasis inhibitors have been clinically established.

As described above, the present inventors have found that cancer metastasis can be inhibited by biochemically inhibiting the function of connexin 26.

Therefore, if such a compound that specifically inhibits the function of connexin 26 is found, the compound is expected to be a cancer metastasis inhibitor. If a cancer metastasis inhibitor is developed, it is expected that the cancer treatment will be improved dramatically by using the cancer metastasis inhibitor in cancer extirpation operations or using the cancer metastasis inhibitor in combination with an anticancer agent..

In view of the aforementioned problem, an object of the present invention is to provide a connexin 26 inhibitor which inhibits cancer metastasis by specifically inhibiting the function of connexin 26, and a cancer metastasis inhibitor utilizing the connexin 26 inhibitor. The inhibitors do not inhibit the function by directly bonding with connexin 26.

### DISCLOSURE OF INVENTION

Based on finding that inhibiting a function of connexin 26 among a connexin family can inhibit cancer metastasis, the present inventors diligently studied to find a novel compound which specifically inhibits the function of connexin 26.

As a result, it was found that a compound having a ring structure and a terminal amide in its molecule (more specifically in a main chain of its molecule) such as an oxirane-containing aliphatic amide specifically inhibits the function of connexin 26. Further, it was found that an unsaturated fatty acid amide inhibits the function of connexin 26. Based on this, the present invention was established.

Also, it was found that oxirane-containing aliphatic amide specifically inhibits connexin 26 without inhibiting connexin 43.

Among connexin families, connexin 43 exists in central nerves or myocardial cells in a large amount. It is considered that decrease in the amount of connexin 43 causes adverse effects such as arrhythmia or the like. Therefore, it was concluded that the oxirane-containing aliphatic amide of the present invention, which does not inhibit the function of connexin 43, can act as a cancer metastasis inhibitor with less side effects against the function of connexin 43. Based on this, the present invention was completed.

That is, to attain the object, the connexin 26 inhibitor of the present invention is a compound inhibiting a function of connexin 26, the compound having a three-membered ring having substituents in cis configuration in its molecule.

In the above description, " Inhibiting the function of connexin 26" means that the function of connexin 26 is inhibited (controlled) by administering the connexin 26 more than before the administration. That is, an amount of connexin 26 decreases, or gap junctions formed by connexin 26 is inhibited, for example.

It is preferable that at least one of the substituents have a functional group having a carbonyl group at an end thereof.

It is preferable that the functional group having the carbonyl group be a primary amide.

It is preferable that the three-membered ring be oxirane.

It is preferable that the connexin 26 inhibitor of the present invention be a compound represented by General Formula (1): 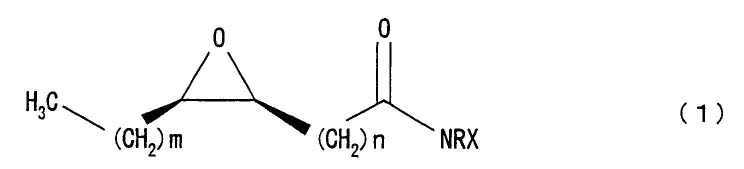 where R is a hydrogen atom or a hydrocarbon group, X is one of a hydrogen atom, a methansulfonyl group, an ethansulfonyl group, an acetyl group, a trifluoroacetyl group, a hydroxyl group, an alkoxy group and an amino group; m is an integer of from 4 to 10; and n is an integer of from 4 to 7.

The connexin 26 inhibitor and cancer metastasis inhibitor of the present invention may be a compound inhibiting a function of connexin 26 and containing unsaturated fatty acid amide having a double bond with cis configuration.

It is preferable that a level of gap junction cell-to-cell communication (GJIC) against connexin 26 be four or smaller for the connexin 26 inhibitor of the present invention.

It is preferable that a level of gap junction cell-to-cell communication (GJIC) against connexin 43 be six or greater for the connexin 26 inhibitor of the present invention.

The connexin 26 inhibitor of the present invention inhibits formation of a gap junction containing connexin 26. Thus, cell-to-cell communication is blocked. As a result, cancer metastasis can be inhibited.

To attain the object, the cancer metastasis inhibitor of the present invention has the connexin 26 inhibitor of the present invention as an active principle.

The cancer metastasis inhibitor of the present invention contains the connexin 26 inhibitor which inhibits cancer metastasis. Therefore, a novel anticancer drug that has not been developed can be provided. This enables inhibiting cancer metastasis, so that efficacy of cancer treatments can be dramatically improved.

For a fuller understanding of the nature and advantages of the invention, reference should be made to the ensuing detailed description taken in conjunction with the accompanying drawings.

### BRIEF DESCRIPTION OF DRAWINGS

Fig. 1 is a view illustrating results of evaluating inhibitory activities against connexin 26 and conenxin 43 in Example 3.
Figs. 2 (a) through (d) are views illustrating results of evaluating cancer metastasis inhibition caused by administering oleamide in Example 4.
Fig. 3 is a graph illustrating how numbers of cancer metastasis foci were changed by administration of oleamide in Example 4.
Fig. 4 is a graph illustrating how numbers of cancer metastasis foci were changed by administrations of oleamide and MI-18 in Example 5.
Fig. 5 is a graph illustrating death rates of mice under administrations of oleamide and MI-18 in Example 5.

### BEST MODE FOR CARRYING OUT THE INVENTION

One embodiment of the present invention is described below. The present invention is not limited to this.

A connexin 26 inhibitor of the present invention specifically inhibits a function of connexin 26, which is one of subtypes of a protein family forming a gap junction. However, the inhibition takes effect not by directly bonding to connexin 26, but by changing a property of a cell membrane so that the function of connexin 26, which is membrane protein, will be inhibited.

In the present invention, it is meant by the wording "inhibiting the function of connexin 26" to inhibit (suppress), by administering the connexin 26 inhibitor of the present invention, the function to which connexin 26 relates, so that the function becomes less active after the administration of the connexin 26 inhibitor. That is, the function to which connexin 26 relates is inhibited to the extent where connexin 26 does not function normally. In the following examples, which demonstrate effectiveness, peritoneal administration of connexin 26 was performed. However, administration is not limited to the peritoneal administration, and may be oral administration or other administration method. Because the effectiveness was shown with the peritoneal administration, there is a high possibility that oral administration will achieve a similar effectiveness.

The connexin 26 of the present invention contains, in its molecule, substituents bonding to a three-membered ring of a cis configuration. The three-membered ring structure is contained in the molecule (more specifically in a main chain of the molecule).

Specific examples of the three-membered ring are rings such as ethylene oxide, aziridine, cyclopropane, and the like. Among these, ethylene oxide (so called, epoxide) is preferable which can be easily produced.

The following describes ethylene oxide as the three-membered ring.

In the present invention, ethylene oxide is cis-oxirane, in which substituents are bonded in the cis configuration. One of the substituents contains a functional group having a carbonyl group at its end.

Examples of the functional group are: amides, hydrazides, carboxylic acids, esters, hydroxamic acids, ketones, aldehydes, and the like. Among these, amides and hydrazides that contain nitrogen are preferable, and primary amides are more preferable. As described later, a terminal amide bond provides higher inhibitory activity against connexin 26.

That is, it is highly preferable that the connexin 26 inhibitor has an oxirane structure of the cis configuration and a terminal amide structure in its molecule (more specifically in a main chain of the molecule).

Each of the substituents of the cis configuration is a hydrocarbon group. One of the substituents contains the functional group having the carboxyl group at its end.

Specific examples of the hydrocarbon group are: straight-chained or branched alkyl groups having a carbon number of 1 through 12, cyclic alkyl groups with a carbon number of 3 through 12, straight-chained or branched alkenyl groups with a carbon number of 2 through 12, cyclic alkenyl groups with a carbon number of 3 through 12, and an aryl groups such as phenyl group and the like. Specific examples of the alkyl groups are: methyl group, ethyl group, propyl group, isopropyl group, butyl group, isobutyl group, t-butyl group, pentyl group, isopentyl group, t-pentyl group, hexyl group, octyl group, cyclopropyl group, cyclobutyl group, cyclopentyl group, cyclohexyl group, and methylcyclohexyl group, and the like. Specific examples of the alkenyl group includes allyl group.

The hydrocarbon group may have an inactive atom or a functional group as long as the connexin 26 inhibitory action is not lost.

The cis-oxirane is preferably a disubstituted oxirane, but may be a trisubstituted or tetrasubstituted oxirane. It has been known that oleamide, which is an amide derived from oleic acid, inhibits a gap junction derived from glial cells (Japanese Laid-Open Patent Publication No. 523696/2001, "*Tokuhyo* 2001-523696").

Thus, the connexin 26 inhibitor of the present invention is preferably an oxirane-containing aliphatic amide derived from a fatty acid. The oxirane-containing aliphatic amide has an oxirane structure (preferably in cis configuration) in its molecule (more specifically, in a main chain of fatty acid), and a terminal carboxyl group of the fatty acid converted into an amide (that is, the terminal carboxyl group is a derivative). In other words, the connexin 26 inhibitor of the present invention is made of a compound having a fatty acid as its main chain, the fatty acid having the oxirane (three-membered ring) structure having substituents in the cis configuration in its molecule (preferably a compound having an amide as the terminal carboxyl group of the fatty acid).

The oxirane-containing aliphatic amide is preferably derived from a straight-chained fatty acid similar to biogenic fatty acid. Specifically, a carbon number of the fatty acid is preferably 12 through 21, more preferably 16 through 20, and most preferably 18 (e.g. oleic acid). The "biogenic fatty acid" is biosynthesized through *in vivo* metabolism and decomposition. That is, the biogenic fatty acid is contained *in vivo* in general. Thus, the connexin 26 inihibitor becomes similar to a biomolecule. Therefore, the connexin 26 inhibitor can be used as a cancer metastasis inhibitor with less side effects.

For the oxirane-containing aliphatic amide, the oxirane structure is not particularly limited. However, the oxirane structure is preferably located between the 9th and 10th carbons. With this arrangement, a higher inhibitory activity against the connexin 26 is attained.

For example, the connexin 26 inhibitor of the present invention is preferably an oxirane-containing aliphatic amide represented by the following general formula (1):

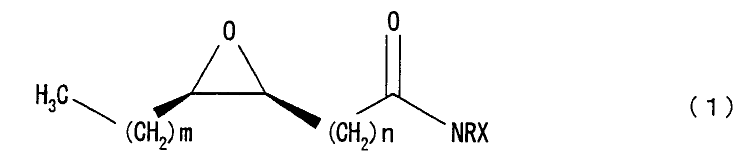

In the general formula (1), if the substituent R is a hydrocarbon group, the hydrocarbon group is one of the hydrocarbon groups exemplified in the above the hydrocarbon groups having the substituents in the cis configuration.

Moreover, in the general formula (1), the substituent X combined with the terminal amide is one of a hydrogen atom, a methansulfonyl group, an ethansulfonyl group, an acetyl group, a trifluoroacety group, a hydroxyl group, an alkoxy group, and an amino group. That is, the substituent X (excluding a hydrogen atom) is a protecting group of a nitrogen atom in the amide group. When hydrazide has an amino group for the substituent X, the amino group may be combined with a substituent or protecting group.

The connexin 26 inhibitor of the present invention may be an unsaturated fatty acid amide having a double bond with cis configuration, such as oleic acid. In this case, as mentioned above, a primary amid is preferable.

As described above, a carbon number of the unsaturated fatty acid is preferably 12 through 21, more preferably 16 through 20, and most preferably 18 (e.g. oleamide). The location of the double bond in the unsaturated fatty acid amide is not particularly limited. However, it is preferably located at the 9th position (between the 9th and 10th carbons counted from the terminal amide carbonyl carbon; Δ 9 cis double bond), thereby enhancing the inhibitory activity against connexin 26.

The unsaturated fatty acid amide may be a straight-chained or a branched fatty acid and contain another functional group in its molecule. Also, the terminal amide may be the functional group having a carbonyl group, as long as the unsaturated fatty acid inhibits the function of the connexin 26.

### (2) Method for Producing Connexin 26 Inhibitor

The oxirane-containing aliphatic amide derived from the fatty acid can be produced in a following manner.

Reactions of converting a carbon-to-carbon double bond (alkene) into oxirane or converting a carboxylic acid into an amide are generally performed. Therefore, the connexin 26 inhibitor containing an oxirane-containing aliphatic amide can be produced easily from a fatty acid containing an alkene and a carboxylic acid in its molecule.

For example, the oxirane-containing aliphatic amide can be produced easily from unsaturated fatty acids widely available in nature. Unsaturated fatty acids are also biosynthesized in vivo. Thus, the oxirane-containing aliphatic amide produced from an unsaturated fatty acid becomes a connexin 26 inhibitor that is more similar to a biomolecule.

The Method for producing connexin 26 inhibitor from the unsaturated fatty acid includes two steps; (i) the step of transforming a double bond of the unsaturated fatty acid into oxirane with a peracid; and (ii) the step of converting carboxylic acid of unsaturated fatty acid into an amide with ammonia.

Since the connexin 26 inhibitor of the present invention contains a three-membered ring of the cis configuration, the unsaturated fatty acid of the cis configuration in the production method is, e.g., any compound selected from sterculic acid, palmitoleic acid, oleic acid, recinoleic acid, petroselinic acid, vaccenic acid, linoleic acid, eleostearic acid, punicic acid, arachidonic acid, icosenoic acid, and eicosapentaenoic acid. However, the selection is not limited to these.

Further, the double bond in the unsaturated fatty acid is preferably located in position 9. With this, the inhibitory activity against the connexin 26 is enhanced.

As described above, the connexin 26 inhibitor can be easily produced. Thus, production of the connexin 26 inhibitor can be established in a short time, by screening a larger number of candidate compounds produced through combinatorial synthesis in accordance with the following method for evaluating the connexin 26 inhibitory activity, so as to select a compound that exhibiting the connexine 26 inhibitory activity.

The above describes producing the connexin 26 inhibitor through the chemical synthesis. However, the connexin 26 inhibitor may be isolated from nature or generated by use of microorganisms.

### (3) Evaluation of Connexin 26 Inhibitory Activity

The following describes a method for evaluating the connexin 26 inhibitory activity.

The connexin 26 inhibitory activity can be evaluated with dye-transfer assay, for example. Specifically, a gap junction level (GJIC score) against the connexin 26 is used for evaluation (c.f. Japanese Laid-Open Patent Publication No. 17184/2001, "*Tokukai* 2001-17184, publication date: January 23, 2001, and M. Mensil et al. Cancer Res. Vol55, 629-639 1995.).

With this method, labelling dye, which can be transferred to outside of cells only through the gap junctions, is introduced into cells. Then the cells are smeared on a monolayer culture or a tissue culture. If gap junctions are formed between (a) the cells (donor cells) to which the dye has been introduced and (b) cells of the monolayer culture or tissue culture (cells adjacent to the donor cells), dye transfer from the donor cells to the adjacent cells can be observed. This method is used for observing cell-to-cell transfer of fluorescent dye under fluorescent microscope or the like.

As described above, the connexin 26 is one of the molecules forming the gap junction. Thus, when a candidate compound having the connexin 26 inhibitory activity inhibits the function of the connexin 26, the gap junction cannot be formed between the cells. As a result, the labeling material that can be transferred only through the gap junction cannot bed transferred between the cells.

Thus, if the fluorescent microscope observation or the like showed that no or less transfer of fluorescent material occurs after administration of the connexin 26 inhibitor, the connexin 26 inhibitor is determined to have the inhibitory activity. Conversely, when the transfer of the labeling material between many cells is observed, the connexin 26 inhibitor is determined to exhibit little inhibitory action.

GJIC score can be calculated as described in following examples. That is, if no dye-coupling occurs with respect to the donor cells, "0" is scored; "1" is scored if dye-coupling from the donor cell to an adjacent receptive cell occurs; and "2" is scored if dye-coupling from the donor cell to a second adjacent cell occurs. In this manner, when dye-coupling to n^{th} adjacent cell from the donor cell occurs, "n" (an integer) is scored. This "n" is the GJIC score.

The gap junction cell-to-cell communication (GJIC) score with respect to connexin 26 is four or smaller preferably, and three or smaller more preferably. When the GJIC score is as such, the function of the connexin 26 can be inhibited certainly.

That is, a gap junction cell-to-cell communication (GJIC) score closer to zero is preferable because it gives higher inhibitory activity against the connexin 26.

The connexin 26 inhibitor of the present invention inhibits at least the function of the connexin 26, and may inhibit connexin of other subtypes.

However, in using the connexin 26 inhibitor as an anticancer agent (cancer metastasis inhibitor), it is preferable that no function of connexin 43 be inhibited. Because connexin 43 is highly specific to myocardial cells, a gap junction formed by the connexin 43 transmits stimuli of muscle contraction to cardiac muscle. Therefore, inhibiting the function of the connexin 43 might cause side effects such as arryhythmia.

Thus, it is preferable that the connexin 26 inhibitor of the present invention have not only the connexin 26 inhibitory action, but also a greater gap junction cell-to-cell communication (GJIC) score. This enables preventing the side effects such as arryhythmia caused by inhibiting the function of the connexin 43.

More specifically, the gap junction cell-to-cell communication (GJIC) score is preferably six or greater, and seven or greater more preferably. With this, the function of the connexin 43 will not be inhibited.

### (4) Cancer Metastasis Inhibitor of Present Invention

The present inventors have found that cancer metastasis can be inhibited by biochemicaly inhibiting the function of the connexin 26 (disclosed in Japanese Laid-Open Patent Publication No. 17184/2001, "*Tokuhyo* 2001/17184", Ito, A. et al.: J. Clin. Invest., 105: 1189-1197, 2000). Thus, it is only necessary for the cancer metastasis inhibitor of the present invention to contain the connexin 26 inhibitor of the present invention.

In using the connexin 26 inhibitor as a cancer metastasis inhibitor, the connexin 26 might be metabolized. In some cases, for example, substitution reaction, addition reaction, or elimination reaction may occur in the molecule of the connexin protein inhibitor, so that a change occurs in the molecule of the compound exhibiting the connexin 26 inhibitory action.

Thus, in the cancer metastasis inhibitor of the present invention the connexin 26 inhibitor encompasses (i) a connexin 26 inhibitor exhibiting cancer metastasis inhibitory activity without being metabolically activated, and (ii) a connexin 26 inhibitor exhibiting cancer metastasis inhibitory activity with being metabolically activated.

Since drugs are foreign materials to living organisms, drugs are designed to have structures similar to biomolecules in drug development. With this, there is a possibility that drugs with less side effects can be developed.

In living organisms, various fatty acids are synthesized such as palmitic acid, stearinic acid, oleic acid, linolic acid, linoleic acid, and arachidonic acid, for example. As described above, the double bond of unsaturated fatty acids can be easily converted into oxirane. Also, fatty acids can be easily converted into amide because fatty acids have a carboxyl group at its end. Further, in living organisms, there is a path to generate acetyl CoA by metabolizing fatty acids. Thus there is a very high possibility that it is possible to provide a cancer metastasis inhibitor that is similar to a biomolecule and thus has less side effects, by designing the molecule of the connexin 26 inhibitor using a fatty acid as its basic structure.

Thus, in using the connexin 26 inhibitor as a cancer metastasis inhibitor, the oxirane-containing aliphatic amide is highly preferable.

Further, the conversion from the double bond to oxirane and the conversion from calboxylic acid to amide can be easily carried out. Therefore, oxirane and the double bond are equivalent, while calboxylic acid and amide are equivalent. Besides this, substitutions among atoms with similar radii (e.g. substitution from hydrogen to fluorine) are also equivalent.

In the cancer metastasis inhibitor of the present invention, the functional group having (i) oxirane of the cis configuration and the functional group having (ii) a carbonyl group at its end encompass not only oxirane and the amide structure, but also groups almost equivalents thereto and easily transformed to oxirane and the amide structure.

In the present invention, it is meant by the wording "including connexin 26 inhibitor" to include any one of (i) the connexin 26 inhibitor of the present invention and (ii) a compound that is *in vivo* metabolized into the connexin 26 inhibitor.

That is, the cancer metastasis inhibitor of the present invention is a drug containing (i) the connexin 26 inhibitor of the present invention, (ii) or a compound having a molecule equivalent to the connexin 26 inhibitor of the present invention, (iii) a compound that is *in vivo* converted to an active metabolite that exhibits cancer metastasis inhibitory action, the active metabolite being the connexin 26 inhibitor of the present invention or a molecule equivalent to the connexin 26 inhibitor of the present invention.

Molecules are recognized stereospecifically in living organisms. In enzymatic reaction for example, one molecular structure of a racemic body can be recognized, whereas another molecular structure thereof cannot. As a result, in the case of a drug of racemic body, one molecule exhibits medicinal activity, whereas the other molecule does not. In some case, the other molecule rather exhibits a side effect.

Thus, the cancer metastasis inhibitor also preferably contains an optically active substance of the connexin 26 inhibitor which exhibits cancer metastasis inhibitory action. Specifically, the connexin 26 inhibitor preferably has an asymmetric three-membered ring structure. That is, in the above production method, the step for transforming the double bond to oxirane may be an asymmetric reaction, so as to inhibit cancer metastasis effectively without causing side effects.

By not only being similar to biomolecules, but also containing the connexin 26 inhibitor of an optically active substance, the cancer metastasis inhibitor can further reduces side effects.

As described in the following examples, the oxirane-containing fatty amide derived from the fatty acid exhibits the inhibitory action against connexin 26, while exhibiting no inhibitory action against connexin 43. That is, the oxirane-containing fatty amide is free from side effects such as arrhythmia or the like caused as a result of the inhibitory action against connexin 43. Thus, the oxirane-containing aliphatic amide is highly preferable to be used as a safe cancer metastasis inhibitor having less side effects.

The cancer metastasis inhibitor may be a prodrug from which the connexin 26 inhibitor or the molecule equivalent thereto is produced *in vivo* as an active metabolite.

In using the connexin 26 inhibitor, the structure of the connexin 26 inhibitor can be modified to improve absorption, distribution, metabolism, and/or excretion *in vivo*.

The cancer metastasis inhibitor may contain the connexin 26 inhibitor with an amount allowing the cancer metastasis inhibitory action in vivo. In using the connexin 26 inhibitor as a cancer metastasis inhibitor, the cancer metastasis inhibitor also contain a common drug addictive, such as an excipient, a binder, or the like with an appropriate amount, in addition to the connexin 26 inhibitor.

It has been known that oleamide, which is an amide derivative of oleic acid, and its derivatives (oleamide and its derivatives are denominated as "oleamides") inhibit gap junctions (Boger, D. L. et al., Proc. Nat. Acad. Sci. USA., 95:4810-4815. 1998.). However, selectivity of inhibitory action of oleamides against the subtypes of connexin has not been examined. The present inventors examined the selectivity of the inhibitory action of oleamides against the subtypes of connexin for the first time.

The examination showed that oleamide exhibited inhibitory action against both connexin 26 and connexin 43.

On the contrary, the oxirane-containing aliphatic amide represented by general formula (1) exhibited strong inhibitory action against connexin 26 as much as oleamide, while exhibiting no inhibitory action against connexin 43. That is, the oxirane-containing aliphatic amide represented by general formula (1) has high selectivity against connexin 26.

It is predicted that the inhibitory action against connexin 43 has adverse effects to central nerves or myocardial cells. Therefore, by using oleamide exhibiting inhibitory action against connexin 43 as a cancer metastasis inhibitor, side effects such as arrhythmia or the like might be caused.

Thus, the oxirane-containing aliphatic amide presented by the general formula (1), which exhibits no inhibitory action against connexin 43 while exhibiting specific inhibitory action against connexin 26, is a cancer metastasis inhibitor with less side effects.

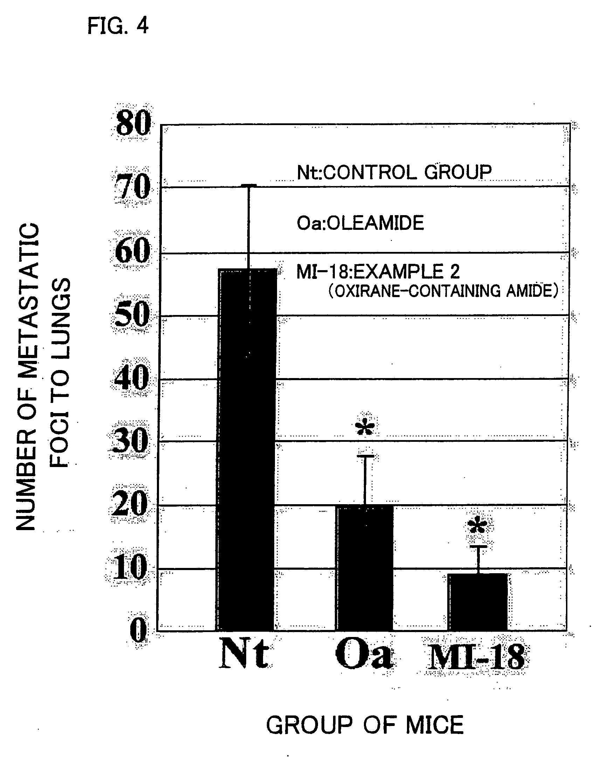

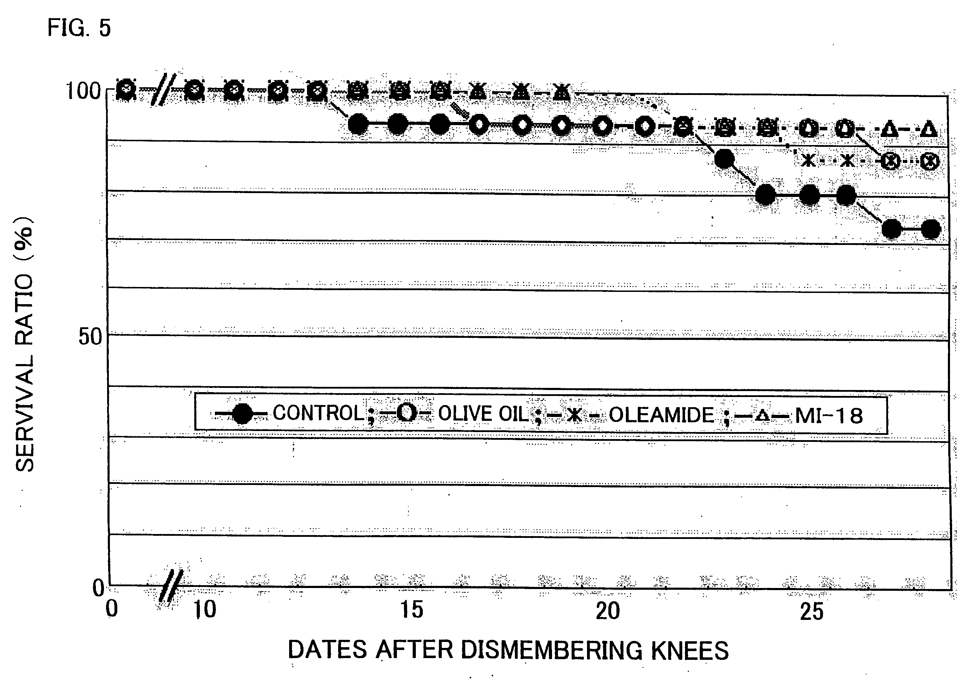

As described above, Boger, D. L. et al., Proc. Nat. Acad. Sci. USA., 95: 4810-4815. 1998. describe that oleamide inhibits the gap junctions. However, Boger does not describe or suggest which subtypes of connexin were inhibited to inhibit the gap junctions. As described in the following Examples, the present inventors examined inhibitory actions of oleamide against connexin 26 and connexin 43. As a result, it became clear that oleamide exhibited inhibitory action against both connexin 26 and connexin 43. Therefore, using oleamide as a cancer metastasis inhibitor might cause side effects such as arrhythmia or the like, due to the inhibitory action of the connexin 43. Therefore, oleamide is considered to be inappropriate as a cancer metastasis inhibitor. However, the following examples showed that even when administering oleamide, death rates due to causes other than cancer were low (refer to Figs. 4 and 5). Thus, oleamide can be sufficiently used as a cancer metastasis inhibitor.

As described in the following Examples, it should be noted in the present invention that the cancer metastasis inhibitor of the present invention inhibits (suppresses) spontaneous metastasis of cancer. For conventional cancer metastasis inhibitors, drug efficacy has been tested (the inhibitory degrees of cancer metastasis have been evaluated) by metastasizing cancer experimentally, not by transplanting cancer cells. That is, cancer cells were injected intravenously, and effectiveness was examined based on cancer metastasis into lungs in two weeks after the administration of cancer metastasis inhibitor. On the other hand, in the following Examples, cancer cells were transplanted, and drug efficacy was examined based on cancer metastasis after the transplantation. Accordingly, the efficacy can be examined under a condition similar to real cancer. That is, the cancer metastasis inhibitor of the present invention inhibits (suppresses) spontaneous metastasis of cancer, which directly reflects cancer metastasis of humans. Thus, the cancer metastasis inhibitor of the present invention is a highly useful drug.

The present invention is not limited to the foregoing embodiment, and modifications can be varied in the scope of the claims. That is, the technical scope of the present invention also includes embodiments obtained by appropriately combining the technical means disclosed in different embodiments.

### [Examples]

In the following Examples, magnetic nuclear resonance (¹H-NMR) spectrum was measured with tetramethylsilane as internal reference material by using a spectrometer JEOL JNM-AL300 (300 MHz). Mass spectroscopy (MS) spectrum was measured according to a direct method using 20eV or 70eV, by using a spectrometer JEOL JNM-LMS300. For column chromatography, Merck Kiesege 160 (70-230 mesh ASTM) was used as an absorbent.

### [Example 1] Production of 9(Z)-Octadecenamide (oleamide)

Under nitrogen atmosphere, oxalyl chroride (0.45ml, 5.3mmol) is dropped into an absolute methylene chloride solution (8.5ml) of 9(Z)-octadecenoic acid (490mg 1.8mmol) cooling the solution with ice. Then the solution was stirred at room temperature for four hours. After the solvent was distilled out and aqueous ammonia (10ml) was added thereto. The solution was stirred at room temperature for 30 minutes. After water (10ml) was added thereto, extraction was performed with ethyl acetate (15ml x 3) added thereto. An organic layer thus obtained was washed with saturated saline (10ml). Next, the organic layer was dried with anhydrous sodium sulfate and then concentrated under reduced pressure, thereby obtaining a crude product. The crude produce was purified using a silica gel column chromatography (hexan: ethyl acetate = 1 : 2). Thereby, octanamide (350mg, 71%) was obtained.
¹H-NMR (CDCl₃, 300MHz): δ 0.88 (3H, t, J=6.4Hz), 1.24 to 1.33 (20H, m), 1.58 to 1.65 (2H, m), 1.99 to 2.02 (4H, m), 2.22 (2H, t, J=7.5 Hz), 5.32 to 5.36 (2H, m).

### [Example 2] Production of 8-[(2S* 3R* )-3-octyloxirane-2-yl] octanamide

Cooling with ice, m-chloro perbenzoic acid (49mg, 0.23mmol) was added to methylene chloride solution (3ml) of oleamide (53mg, 0.19mmol) obtained in Example 1, thereby preparing a solution. The solution was stirred at room temperature for three hours. Cooling with ice, saturated sodium thiosulfate aqueous solution (3ml) was also added thereto, and then extraction was performed with ethyl acetate (5ml x 3). An organic layer thus obtained was washed with saturated saline (10ml). Next, the organic layer was dried with anhydrous sodium sulfate sulfate and then concentrated under reduced pressure, thereby obtaining a crude product. The crude product was purified using silica gel column chromatography (ethyl acetate). In this way, oxirane-containing amide (MI-18): 8-[(2S*3R*)-3-octyloxirane-2-yl] octanamide (54 mg, 96%) was obtained. This oxirane-containing amide was one of the compounds contained in the general formula (1).

### [Example 3] Test of Inhibitory Action against Connexin 26

### (1) Cell Strain and Cell Culture

Human uterocervical squamaous carcinoma cells (HeLa cells) were purchased from American type culture collection (Manassas, VA). A HeLa subclone (HeLa Cx26), which expressed rat Cx26 stably, was obtained previously (Duflot - Dancer et al., Oncogen, 1997). All cells were incubated in Dulbecco's modified eagle's medium (DMEM) with 10 % fetal calf serum (FCS).

### (2) Plasmid Construction and Transfection

A pCX4bsr vector was derived from the pCXbsr vector (T Akagi., et al., 2000, Proc. Natl. Acad. Sci. USA) with minor modification. The pCX4bsr vector was kindly given by Dr. T Akagi (Osaka Bioscience Institute, Osaka, Japan). The pBluescriptII SK + plasmid vector contained a cDNA fragment of an entire coding region of rat Cx43 between the Spel and ClaI sites, and was constructed previously (Y. Omori., et al., 1998, Int. J. Cancer). The Cx43cDNA was excised by digesting NotI and HincII, and being ligated with the pCX4bsr where NotI and HincII were digested. HeLa cells were transfected with the resulting pCX4bsr-Cx43 vector by using the TransFast transfection regent. After transfection, cells were selected by resistance to blasticidin (3 µ g/ml) for three weeks to obtain single colonies. One of the stably transformed clones (HeLa-Cx43) was used for dye-transfer assay.

### (3) Dye-Transfer Assay

A day before assay, HeLa-Cx26 or HeLa-Cx43 was suspended in DMEM containing 10% FCS. Both cells were cultured either on 6-well plates or coverslips at the bottom of 6-well plates. The former cells were used as donor cells, and the latter cells as recipient cells. Initial plating was adjusted to yield subconfluent monolayers next day. Next day, calcein-AM and DiI were added to the culture of donor cells at concentrations of 4 µ M and 10 µ M, respectively, and incubated for one hour. Double-labeled donor cells were trypsinized and washed three times with phosphate-buffered saline. A thousand single donor cells suspended in DMEM containing 10 % FCS were gently overlaid on unlabeled recipient cells. It took about one hour for donor cells to settle over a monolayer, and about 1.5 hours to begin to exhibit dye-coupling. One hour after donor cells were planted, agents were added to the culture at concentration indicated in Table 1, in order to evaluate inhibitory effects of agents on dye-coupling. For control, ethanol was added to the culture at 1 : 1000 dilution. After culturing them for one hour, the coverslips were removed from the 6-well plates and observed with a confocal laser scanning microscope (LSM510).

Cells containing the gap junction-impermeable DiI (red fluorescence) were identified as donor cells. Transfer of gap junction-permeable calcein from DiI-positive donor cells into DiI-negative recipient cells was regarded as dye-coupling.

The level of gap junction cell-to-cell communication (GJIC) was scored by the following methods.

Donor cells were scored as 0 for no dye-coupling, as 1 for dye-coupling from a donor cell to an adjacent recipient cell, and as 2 or 3 when calcein transferred to the adjacent recipient cell further transfers to a second or a third adjacent cell, subsequently, in a direction away from the donor cell. Up to "8" is set because it was difficult to evaluate dye transfer from the donor cell to cells which are eight or more cells away therefrom. Per factor, at least 25 donor cells were used for scoring under a condition that the donor was a single cell that was at least 10 cells away from another single donor. The experiments were repeated three times and mean and standard deviation of score counts were calculated for each factor. For comparison, an unpaired Student's test was done, and a P value < 0.01 was considered to be significant difference.

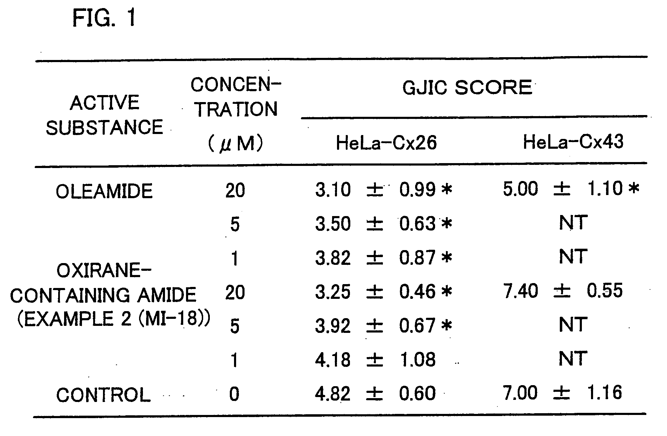

Fig. 1 illustrates the result of evaluating inhibitory activity of oleamide produced in Example 1 and the oxirane-containing amide produced in Example 2 against connexin 26 and connexin 43. As illustrated in Fig. 1, it was confirmed that both of the compounds exhibits inhibitory activity against connexin 26, and that oxirane-containing amide specifically inhibits only connexin 26.

### [Example 4] Test of Cancer Metastasis Inhibitory Action of Connexin 26 Inhibitor-1

After subcutaneously inoculating 1 x 10⁵ BL6 cells (substrains of B16 mouse melanoma cells) to a planter of each C57B1/6 mouse, the mice were classified into three groups.
1) Vehicle administration group: peritoneal injection of olive oil 0.2ml twice a day (morning and evening) on consecutive days;
2) Oleamide (25mg/kg/day) administration group: peritoneal injection of olive oil 0.2ml dissolved with oleamide at concentration of 25mg/kg/day twice a day (morning and evening) on consecutive days; and
3) Oleamide (50mg/kg/day) administration group: olive oil 0.2ml dissolved with oleamide at concentration of 50 mg/kg/day twice a day (morning and evening) on consecutive days.

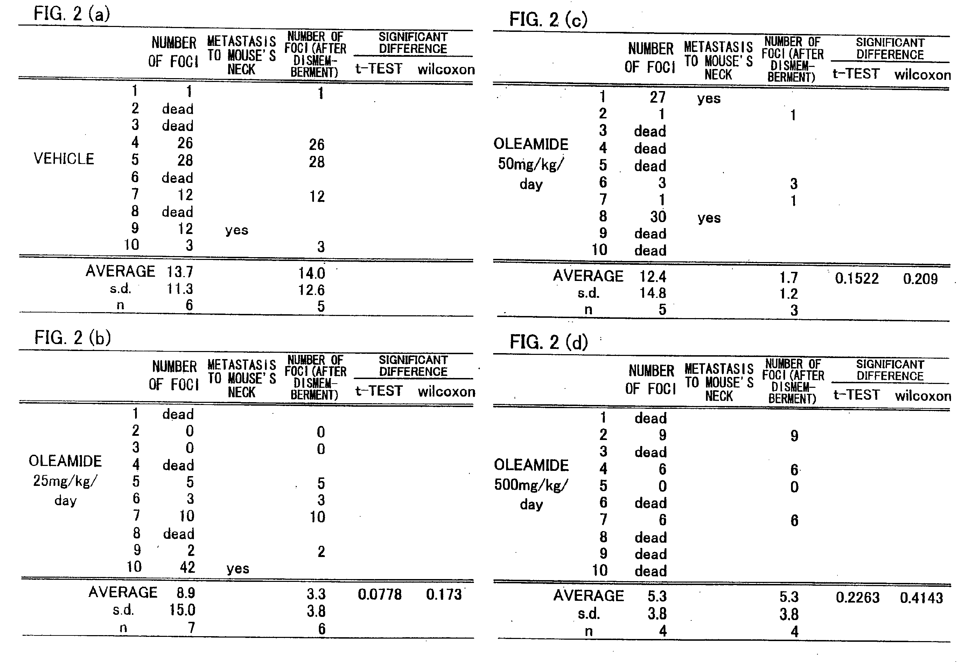

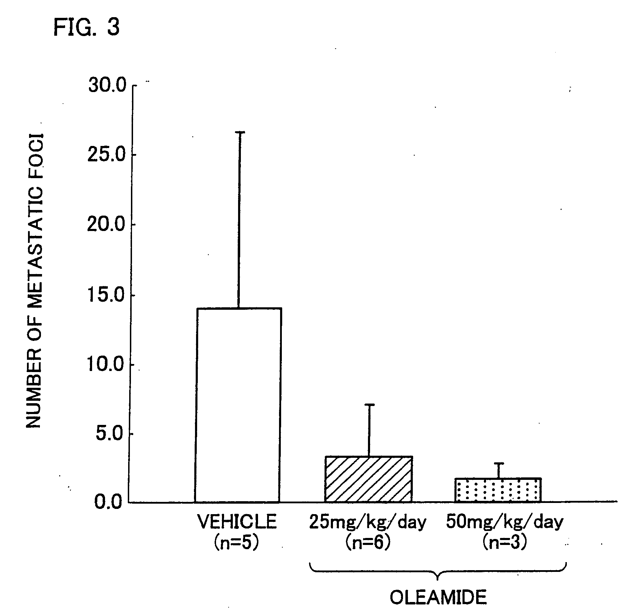

The mice were treated in the above manners on consecutive days after the BL6 cells inoculation. Then, on about 18th day, tumors (about six mm in diameter) formed at inoculation points were removed by dismembering at knee joints. Then the mice were treated in the above manners for another two days after the dismembering. After that, the mice were fed without the treatments. At four weeks after the dismembering at the knee joints, the mice were euthanized, and metastasized tubercles in both left and right lungs (the number of focuses) were counted visually (refer to Fig. 2 (a) through (c) and Fig. 3). Cancer metastasis inhibitory action was evaluated using 10 mice for each group. It was found that the groups of oleamide administration had fewer metastasized tubercles and significantly exhibited cancer metastasis inhibitory action compared with the group having no administration.

The same experiment was performed on oleamide (500mg/kg/day) administration group, and the similar result was obtained (refer to Fig. 2 (d)).

### [Example 5] Test of Cancer Metastasis Inhibitory Action of Connexin 26 Inhibitors-2

After subcutaneously inoculating 1 x 10⁵ BL6 cells (substrains of B16 mouse melanoma cells) to a planter of each C57B1/6 mouse, the mice were classified into three groups.
1) Control mice (Nt) with no treatment but transplanting polyoma cells;
2) Oleamide (25mg/kg/day) administration group: peritoneal injection of olive oil 0.2ml dissolved with oleamide at concentration of 25mg/kg/day once a day on consecutive days; and
3) MI-18 (10mg/kg/day) administration group: olive oil 0.2ml dissolved with MI-18 at concentration of 10mg/kg/day once a day on consecutive days.

The mice were treated in the above manners every day after the BL6 cells inoculation. Then, on about 18th day, tumors (about eight mm radical) formed at the injected points were removed by dismembering at knee joints. Then the mice were treated in the above manners for another two days after the dismembering. After that, mice were fed without the treatments. At four weeks after dismembering at the knee joints, the mice were euthanized, and metastasized tubercles in both left and right lungs (the number of focuses) were counted visually (refer to Fig. 2 (a) through (c) and Fig. 3). Cancer metastasis inhibitory action was evaluated using 15 mice for each group. It was found that the groups of oleamide and MI-18 administrations had fewer metastasized tubercles and significantly exhibited cancer metastasis inhibitory activity, compared to the group having no injection. As illustrated in Fig. 5, no mice died due to other causes but cancer in approximately two months, even though the materials were administered every day. Therefore, these materials are highly safe. Thus, it is highly possible that these materials can be used as cancer metastasis inhibitors (anticancer metastasis drugs) with less (or no) side effects.

The invention being thus described, it will be obvious that the same way be varied in many ways. Such variations are not to be regarded as a departure from the spirit and scope of the invention, and all such modifications as would be obvious to one skilled in the art are intended to be included within the scope of the following claims.

### INDUSTRIAL APPLICABILITY

As described above, according to the present invention, it is possible to provide a connexin 26 inhibitor which specifically inhibits a function of connexin 26. Further, the present invention provides a cancer metastasis inhibitor having the connexin 26 inhibitor of the present invention as an active principle.

The cancer metastasis inhibitor of the present invention specifically inhibits the function of connexin 26, without inhibiting the function of connexin 43. Therefore, it is possible to provide a safe cancer metastasis inhibitor with less side effects such as arrhythmia or the like. Thus, cancer metastasis is inhibited, so that the efficacy of cancer treatments can be dramatically improved.

## Claims

1. A cancer metastasis inhibitor, comprising:
a compound inhibiting a function of connexin 26,
the compound having a three-membered ring having substituents in cis configuration in its molecule.

2. The cancer metastasis inhibitor according to claim 1, wherein the three-membered ring is located in a main chain made of a fatty acid.

3. The cancer metastasis inhibitor according to claim 2, wherein the fatty acid is biogenic.

4. The cancer metastasis inhibitor according to any one of claims 1 through 3, wherein at least one of the substituents has a functional group having a carbonyl group at an end thereof.

5. The cancer metastasis inhibitor according to claim 4, wherein the functional group having the carbonyl group is a primary amide.

6. The cancer metastasis inhibitor according to any one of claims 1 through 5, wherein the three-membered ring is oxirane. connexine 26 inhibitor.

7. The cancer metastasis inhibitor according to any one of claims 1 through 6, represented by General Formula (1): 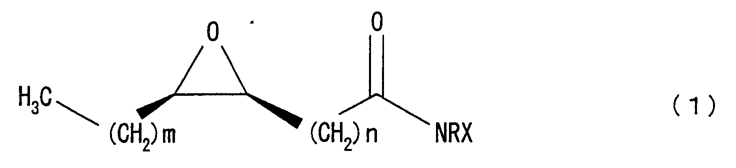 where R is a hydrogen atom or a hydrocarbon group, X is one of a hydrogen atom, a methansulfonyl group, an ethansulfonyl group, an acetyl group, a trifluoroacetyl group, a hydroxyl group, an alkoxy group and an amino group; m is an integer of from 4 to 10; and n is an integer of from 4 to 7.

8. The cancer metastasis inhibitor according to any one of claims 1 through 7, wherein a level of gap junction cell-to-cell communication (GJIC) against connexin 26 is four or smaller.

9. The cancer metastasis inhibitor according to any one of claims 1 through 8, inhibiting no function of connexin 43.

10. The cancer metastasis inhibitor according to clam 9, wherein a level of gap junction cell-to-cell communication (GJIC) against connexin 43 is six or greater.

11. A connexin 26 inhibitor, being a compound inhibiting a function of connexin 26, and having, in its molecule, a main chain made of a fatty acid having a three-membered ring having substituents in cis configuration.

12. The connexin 26 inhibitor according to claim 11, wherein a terminal carboxyl group of the fatty acid is an amide.

13. A connexin 26 inhibitor, being a compound inhibiting a function of connexin 26 and comprising an unsaturated fatty acid amide having a double bond with cis configuration.
